# EUROPÄISCHE PATENTSCHRIFT

(11) **EP 0 953 632 B1**
(45) Veröffentlichungstag und Bekanntmachung des Hinweises auf die Patenterteilung: **05.09.2007**
(21) Anmeldenummer: 99810116.6
(22) Anmeldetag: 11.02.1999
(51) Int. Cl.: C12N 5/00, G01N 33/50

(54) **Verwendung von Nanoemulsionen zur Bestimmung der Biokompatibilität von lipophilen Stoffen im Zellkultur-Test und dafür geeignete Nanoemulsionen**
Use of nanoemulsions for determination of the biocompatibility of lipophilic materials in cell culture test and nanoemulsions suitable therefor
Utilisation des nanoémulsions pour la détermination de la biocompatibilité des matériaux lipophiliques dans les tests des culture cellulaire et nanoémulsions appropriées

(30) Priorität: 30.03.1998 CH 74898
(43) Veröffentlichungstag der Anmeldung: 03.11.1999
(73) Patentinhaber: Mibelle AG Cosmetics, 5033 Buchs (CH)
(72) Erfinder: Zülli, Fred, Dr., 5024 Küttigen (CH); Suter, Franz, 5312 Döttingen (CH)
(74) Vertreter: Rottmann, Maximilian

(56) Entgegenhaltungen:
- EP-A- 0 406 162
- WO-A-89/01027
- DE-A- 19 522 693
- ZULLI, F. ET AL: "Preparation and properties of small nanoparticles for skin and hair care" SOFW J. (1997), 123(13), 880,883-885 CODEN: SOFJEE;ISSN: 0942-7694, XP002079030
- HOFFMANN F ET AL: "Preparation, characterization and cytotoxicity of methylmethacrylate copolymer nanoparticles with a permanent positive surface charge." INTERNATIONAL JOURNAL OF PHARMACEUTICS (AMSTERDAM) 157 (2). 1997. 189-198. ISSN: 0378-5173, XP002079031

## Beschreibung

Die Erfindung betrifft:
- die in den Ansprüchen 1 bis 9 umschriebenen Verwendungen von Nanoemulsionen im Zellkultur-Test zur gezielten und reproduzierbaren Einführung von lipophilen Stoffen in die Zellen.

Nanoemulsionen, auch Nanopartikel genannt, bestehen bekanntlich aus Ölpartikeln, die an ihrer Oberfläche mit einem amphoteren Emulgator besetzt sind, in wässeriger Emulsion. Sie lassen sich beispielsweise herstellen durch Vermischen von Triglyceriden oder Fettsäureestern in wässeriger Phase in einem Hochdruckhomogenisator (EP 0 406 162 B1 - H. G. Weder).

Sie wurden bisher eingesetzt für die Herstellung pharmazeutischer und/oder kosmetischer Zubereitungen, ferner für die Herstellung von Nährstofflösungen für Zellkulturen, in denen die Nanoemulsion als Energielieferant dient (EP 0 406 162 B1 - H. G. Weder). SÖFW Journal, 123 (13), 1997, 880, 883-5 offenbart die verwendung von Nanoemulsionen in der kosmetik.

Die Zellkulturtechnik ist ein biologisches System mit einer sehr breiten Anwendung. Die kontrollierte Züchtung dieser Zellen wird vor allem in der Immunologie, der Biotechnologie, der Toxikologie, der Gentechnologie und der Zellbiologie eingesetzt. In allen diesen Applikationen ist die Interaktion von Stoffen mit den Zellen von zentraler Bedeutung. Der Austausch von biochemischen Stoffen findet im Kulturmedium statt, das aus einer ganzen Reihe von verschieden Stoffen aufgebaut ist. Um ideale Bedingungen für die Zellen zu schaffen, wird dem Kulturmedium meistens ein Blutserum zugegeben. Die Komplementation des Mediums mit Serum weist jedoch für viele Anwendungen grosse Nachteile auf, da das Serum eine undefinierte Zusammensetzung aufweist, teuer ist und unerwünschte Komponenten wie Viren, Prionen und Keime, enthalten kann. Die Entwicklung von sogenannten serumfreien Medien für eine Vielzahl von Applikationen ist daher von besonderer Wichtigkeit.

Eine generelle Schwierigkeit beim Umgang mit Zellkulturen besteht darin, wasserunlösliche Stoffe einzusetzen. Lipophile Stoffe wurden wenn möglich in Alkohol oder Dimethylsulfoxid gelöst und so dem Kulturmedium zugeführt. Dabei entstanden jedoch undefinierte Dispersionen dieser Stoffe mit einer kleinen Bioverfügbarkeit. Die Verwendung von Lösungsvermittlern, wie Tween 20, ergab unstabile und stark toxische Emulsionen. Daher standen bisher keine zuverlässigen und einfachen Methoden zur Verfügung:
- für die Bestimmung der Biokompatilität von Lipiden im Zellkultur-Test;
- für die Bestimmung der Toxizität und/oder der Genotoxizität von lipophilen Stoffen im Zellkultur-Test;
- für die Biotransformation von lipophilen Stoffen mit Zellkulturen;
- für die Förderung der Zelldifferenzierung von primären Zellkulturen;
- für die Trennung von Zellen von nichtinkorporierten Lipiden;
- zur Adaption von Zellen aus einem Vollmedium an ein serumfreies Medium;
- für die Komplementierung von Zellkulturmedien mit lipophilen Stoffen, welche das Wachstum der Zellen fördern;
- für die Komplementierung von Zellkulturmedien mit lipophilen Stoffen, welche die Biosynthese von erwünschten Stoffen in den Zellen fördern; sowie
- für die gezielte und reproduzierbare Einführung von lipophilen Stoffen in die Zellen von Zellkulturen.

Aufgabe der vorliegenden Erfindung ist es nun, derartige Methoden zu schaffen, und weiter für die Verwendung in solchen Methoden geeignete neue Nanoemulsionen zur Verfügung zu stellen.

Diese Aufgabe wird erfindungsgemäss gelöst durch die Verwendung von Nanoemulsionen gemäss den Ansprüchen 1 bis 9.

Die transparenten Nanoemulsionen erleichtern die visuelle Kontrolle der Zellkulturen. Zudem lassen sich Dispersionen mit einer kleinen durchschnittlichen Partikelgrösse besser keimfrei filtrieren (100 nm).

Nanoemulsionen sind sehr stabil und können monatelang im Kühlschrank aufbewahrt werden, bevor sie zu den Zellkulturen gegeben werden.

Zweckmässigerweise weisen die Ölpartikel der Nanoemulsion einen Durchmesser von weniger als 100 nm, vorzugsweise von weniger als 40 nm, auf, und die Besetzung der Ölpartikel mit dem amphoteren Emulgator an ihrer Oberfläche ist zweckmässig einlagig.

Zweckmässigerweise weisen die Nanoelulsionen ein negatives Zeta-Potential auf, vorzugsweise zwischen -10 mV und -50 mV, insbesondere zwischen -30 mV und -40 mV. Für spezielle Anwendungen können auch Nanoemulsionen mit einem positiven Zeta-Potential von Vorteil sein. Solche kationischen Nanoemulsionen können beispielsweise durch Zugabe eines C8- bis C22-Alkylamins erhalten werden.

Zweckmässigerweise enthalten die Nanoemulsionen pro Gewichtsteil Öl, 0,65 bis 0,75 Gewichtsteile, des amphoteren Emulgators. Allgemein gilt, dass der Durchmesser der Ölpartikel mit abnehmendem Anteil des amphoteren Emulgators zunimmt.

Zweckmässigerweise weist der amphotere Emulgator selbst eine geringe Toxizität auf Zellkulturen auf. Vorzugsweise wird Lecithin eingesetzt.

Zweckmässigerweise weist auch die Ölphase selbst eine geringe Toxizität auf Zellkulturen auf, vorzugsweise eine derartige Toxizität, dass deren Wachstum im Vergleich zu einer Referenzkultur ohne Ölkomponente um höchstens 20 % reduziert wird.

Besonders geeignet sind hierfür natürliche Triglyceride. Vorzugsweise setzt man natürliche Triglyceride der Fettsäure C 18:1 (Ölsäure) und/oder der Fettsäure C 18:2 (Linolsäure) ein. Diese besitzen eine sehr niedrige Toxizität und eignen sich besonders gut als Lösungsmittel für die zu untersuchenden lipophilen Substanzen.

Nanoemulsionen können durch Hochdruckhomogenisation von Vormischungen der Komponenten hergestellt werden. Vorzugsweise werden dabei Lecithin zu Öl Verhältnisse gewählt, die 0,65 bis 0,75 sind. Von Vorteil ist weiterhin, wenn die Nanoemulsionen einen kleinen Partikeldurchmesser aufweisen. Gelingt es Öl in Wasser Emulsionen herzustellen, deren Öltröpfchen kleiner als 70 nm sind, so ergeben sich transparente Nanoemulsionen. Diese transparenten Nanoemulsionen erleichtern die visuelle Kontrolle der Zellkulturen. Zudem lassen sich Dispersionen mit einer kleinen durchschnittlichen Partikelgrösse leicht keimfrei filtrieren (100 nm).

Nanoemulsionen sind sehr stabil und können monatelang im Kühlschrank aufbewahrt werden, bevor sie zu den Zellkulturen gegeben werden.

Bevorzugte Ausführungsformen der Erfindung werden in den nachstehenden Beispielen und den beigefügten Zeichnungen erläutert.

### Beispiel 1 (nur zur Information)

### Behandlung von TK6-Lymphoblastoid-Zellen mit einer Nanoemulsion mit einer geringen Toxizität

TK6-Zellen wurden in RPMI-1640-Medium gezüchtet, das mit 2 mmol Glutamin, 5 % Gentamycin und 10 % Pferde-Serum komplementiert wurde. Die Zellsuspension wurde alle 2 bis 3 Tage in neue Zellkulturflaschen überimpft. Die Anzahl der Zellen wurde mit Hilfe einer Neubauer Kammer bestimmt. Der Einfluss der Nanoemulsion 1 und der leeren Lecithin-Partikel (Liposomen) auf das Zellwachstum wurde nach 2 Tagen bei verschiedenen Konzentrationen bestimmt.

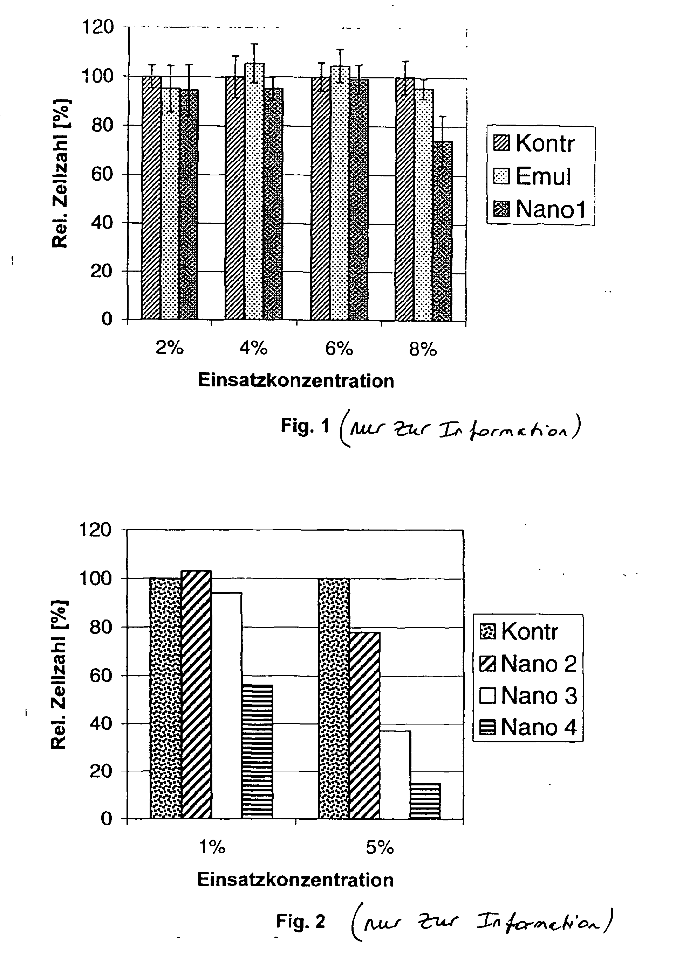

Figur 1 zeigt die relativen Zellzahlen bezogen auf nicht behandelte Zellen ("Kontr" ist 100 %). Die Nanoemulsion 1 ("Nano 1") zeigt bis zu einer Konzentration von 6 % keinen Einfluss auf das Wachstum der Zellen. Der Emulgator ("Emul") (gleiche Lecithin- Konzentration wie Nanoemulsion 1) zeigt sogar bis zu einer Einsatzkonzentration von 8 % keinen Einfluss auf die relative Zellzahl.

Figur 2 zeigt, dass Nanoemulsionen mit anderen Ölkomponenten wesentlich toxischer sind als das Öl in Nanoemulsion 1 bei gleicher Konzentration und gleichem Emulgator. Die Ölkomponenten in Figur 2 sind Sonnenblumenkernenöl ("Nano 2"), hydriertes Erdnussöl ("Nano 3") und ein gesättigtes C 8/C 10-Triglycerid ("Nano 4").

### Zusammensetzung der Nanoemulsion 1

| | |
|---|---|
| Lecithin | 0,6 % |
| Triglycerid | 1,0 % |
| davon: | 90 % C 18:1 und 10 % C 18:2 |
| Partikeldurchmesser | 41 nm |

### Zusammensetzung der Nanoemulsion 2

| | |
|---|---|
| Lecithin | 0,6 % |
| Sonnenblumenkernenöl | 1,0 % |
| Partikeldurchmesser | 45 nm |

### Zusammensetzung der Nanoemulsion 3

| | |
|---|---|
| Lecithin | 0,6 % |
| Hydriertes Erdnussöl | 1,0 % |
| Partikeldurchmesser | 50 nm |

### Zusammensetzung der Nanoemulsion 4

| | |
|---|---|
| Lecithin | 0,6 % |
| Caprylsäure-/Caprinsäure-Triglycerid | 1,0 % |
| Partikeldurchmesser | 45 nm |

### Beispiel 2 (nur zur Information)

### Gezielte Einführung eines UV-Filters in TK6-Lymphoblastoid-Zellen mittels einer Nanoemulsion

TK6-Zellen wurden in RPMI-1640-Medium gezüchtet, das mit 2 mmol Glutamin, 5 % Gentamyzin und 10 % Pferde-Serum komplementiert wurde. Zur Zellsuspension (7 x 10⁵ Zellen/ml) wurden 1 %, 5 % und 10 % der Nanoemulsion 5 gegeben. Nach einer Inkubationszeit von 10 Minuten wurden jeweils 10 ml der Kultur zentrifugiert und die Zellen 2mal mit 10 ml Puffer gewaschen. Die Menge des UV-Filters in den Zellen wurde durch Extraktion mit Chloroform und Messung der Absorption bestimmt (Tabelle 1). Mit der Nanoemulsion 5 konnten definierte Mengen an UV-Filter in die Zellen transportiert werden.

**Tabelle 1**

| | |
|---|---|
| 1 % Nanoemulsion 5 | 10 µg UV-Filter/7 x 10⁶ Zellen |
| 5 % Nanoemulsion 5 | 39 µg UV-Filter/7 x 10⁶ Zellen |
| 10 % Nanoemulsion 5 | 51 µg UV-Filter/7 x 10⁶ Zellen |

Die Aufname der Nanopartikel in die Zellen hängt von der Zeit ab.

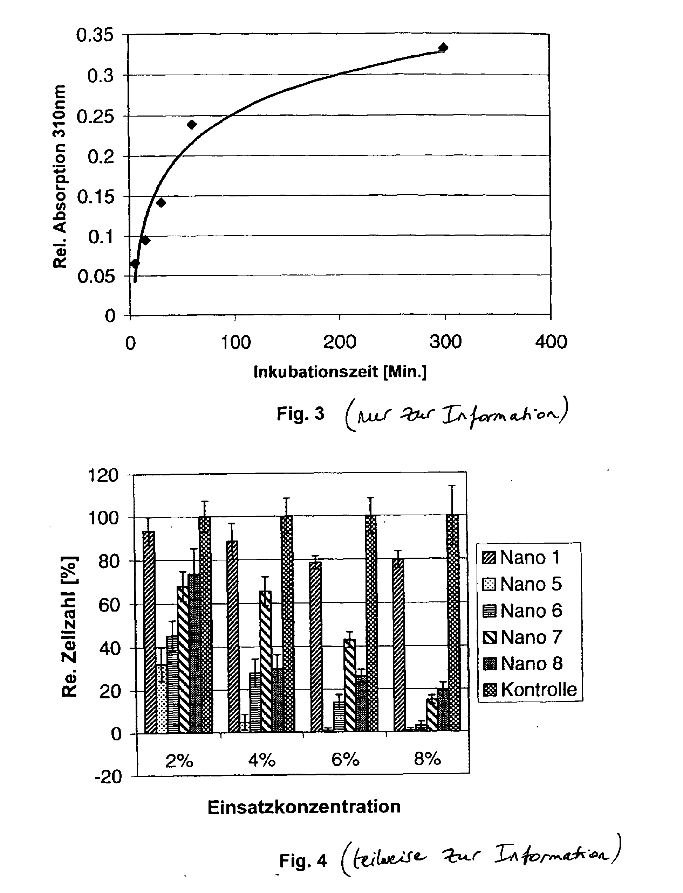

Figur 3 zeigt den zeitlichen Verlauf der Aufnahme von UV-Filtern in die Zellen. 2 x 10⁵ Zellen/ml wurden mit 2 % Nanoemulsion 5 inkubiert. Nach 5, 15, 30, 60 und 300 Minuten wurden jeweils 10 ml der Zellkultur zentrifugiert. Die Zellen wurden 2 mal mit 10 ml Puffer gewaschen und dann mit Chloroform extrahiert. Die relativen Konzentrationen des UV-Filters in den verschiedenen Extraktionen sind in Figur 3 gezeigt.

Im Gegensatz dazu ist die Aufnahme des UV-Filters Isoamyl-methoxyzinnamat in TK6-Zellen aus einem Medium mit einem Zusatz von 2 % einer 0,5%-igen alkoholischen Lösung des UV-Filters völlig undefiniert, da die Zellen nicht richtig von der UV-Filter-Dispersion getrennt werden können (Tabelle 2).

**Tabelle 2**

| Inkubationszeit | Rel. Absorption 310 nm |
|---|---|
| 5 min | 1,0 |
| 5 min | 1,1 |
| 30 min | 1,0 |
| 60 min | 0,9 |
| 240 min | 1,0 |
| Kontrolle ohne Zellen | 0,7 |

### Zusammensetzung der Nanoemulsion 5

| | |
|---|---|
| Lecithin | 0,6 % |
| Triglycerid | 0,5 % |
| davon: | 90 % C 18:1 und 10 % C 18:2 |
| Isoamyl-methoxyzinnamat (4-(4-Methoxyphenyl)-2-propionsäure-3-methylbutyl-ester) | 0,5 % |
| Partikeldurchmesser | 49 nm |

### Beispiel 3

### Gezielte Einführung von UV-Filtern in Fibroblasten und TK6-Lymphoblastoid-Zellen mittels einer Nanoemulsion

Die TK6-Zellen wurden wie in Beispiel 1 beschrieben gezüchtet. Die Fibroblasten (Normal Human Dermal Fibroblasts) wurden in serumfreien Medium (PromoCell) gezüchtet. Zu den Kulturen wurden 2 % Nanoemulsion 6 gegeben und nach einer Stunde die aufgenommene Menge an UV-Filter analog Beispiel 2 bestimmt (Tabelle 3).

### Zusammensetzung der Nanoemulsion 6

| | |
|---|---|
| Lecithin | 0,6 % |
| Triglycerid | 0,9 % |
| davon: | 90 % C 18:1 und 10 % C 18:2 |
| Butyl-methoxydibenzoyl-methan (1-(4-Methoxyphenyl)-3-(4-tert-butyl-phenyl)-3-hydroxy-2-propen-1-on) | 0,1 % |
| Partikeldurchmesser | 50 nm |

**Tabelle 3**

| | |
|---|---|
| TK6-Lymphoblastoid-Zellen | Fibroblasten (NHDF) |
| Menge UV-Filter | Menge UV-Filter |
| 1,43 µg UV-Filter/2,0 10⁶ Zellen 1,15 µg UV-Filter/1,0 10⁵ Zellen | |

### Beispiel 4 (teilweise zur Information)

### Bestimmung der Toxizität von UV-Filtern in TK6-Lymphoblastoid-Zellen

Die TK6-Zellen wurden in RPMI-1640-Medium gezüchtet analog Beispiel 1. Die Anzahl der Zellen wurde mit Hilfe einer Neubauer Kammer bestimmt. Der Einfluss der Nanoemulsionen 5, 6, 7 und 8 mit eingekapselten UV-Filtern und der Nanoemulsion 1 ohne UV-Filter auf die Zellen wurde nach 2 Tagen bei verschiedenen Konzentrationen bestimmt. Figur 4 zeigt die relativen Zellzahlen bezogen auf nicht behandelte Zellen (Kontrolle ist 100 %). Die Toxizität der einzelnen UV-Filter ist unterschiedlich und hängt von der verwendeten Konzentration ab.

### Zusammensetzung der Nanoemulsion 7

| | |
|---|---|
| Lecithin | 0,6 % |
| Triglycerid | 0,9 % |
| davon: | 90 % C 18:1 und 10 % C 18:2 |
| Isoamyl-methoxyzinnamat (4-(4-Methoxyphenyl)-2-propionsäure-3-methylbutyl-ester) | 0,1 % |
| Partikeldurchmesser: | 60 nm |

### Zusammensetzung der Nanoemulsion 8

| | |
|---|---|
| Lecithin | 0,6 % |
| Triglycerid | 0,9% |
| davon: | 90 % C 18:1 und 10 % C 18:2 |
| Benzophenon (2-Hydroxy-4-methoxy-benzophenon) | 0,1 % |
| Partikeldurchmesser | 60 nm |

### Beispiel 5 (teilweise zur Information)

### Bestimmung der Toxizität von UV-Filtern, die einer UV-Bestrahlung ausgesetzt wurden

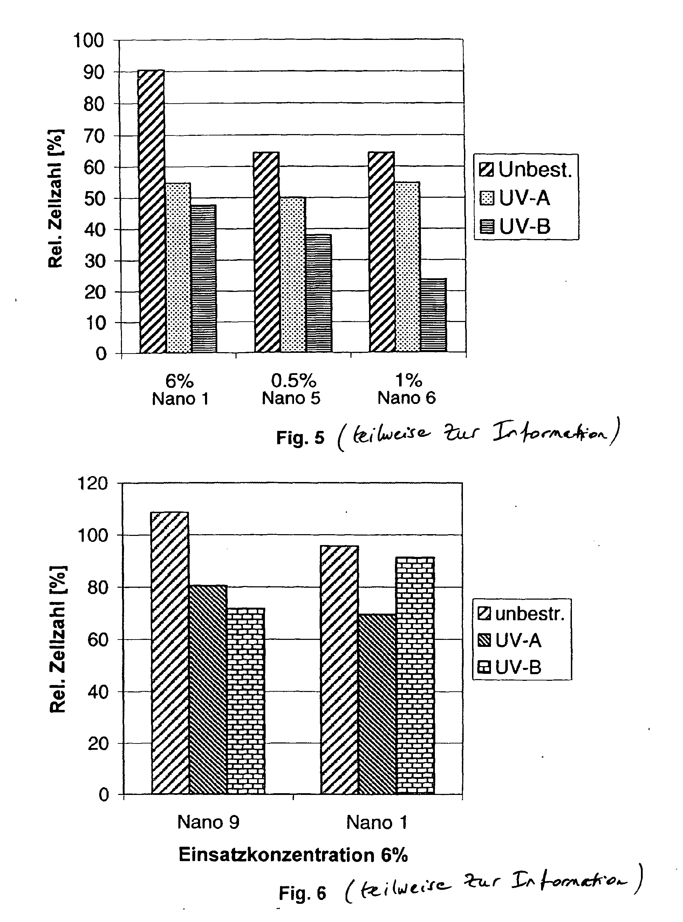

TK6-Zellen wurden in RPMI-1640-Medium gezüchtet analog Beispiel 1. Die Anzahl der Zellen wurde mit Hilfe einer Neubauer-Kammer bestimmt. Der Einfluss der Nanoemulsion 5 und 6 mit eingekapselten UV-Filtern und der Nanoemulsion 1 ohne UV- Filter auf die Zellen wurde vor und nach einer UV-A- und einer UV-B-Bestrahlung der Nanoemulsionen untersucht (Figur 5).

### Beispiel 6 (teilweise zur Information)

### Bestimmung der Toxizität von kosmetischen Lipiden, die einer UV-Bestrahlung ausgesetzt wurden

TK6-Zellen wurden in RPMI-1640-Medium gezüchtet analog Beispiel 1. Die Anzahl der Zellen wurde mit Hilfe einer Neubauer-Kammer bestimmt. Der Einfluss der Nanoemulsion 9 mit eingekapselten mehrfach ungesättigten Fettsäuren (Linolensäure) und der Nanoemulsion 1 auf die Zellen wurde vor und nach einer UV-A- und einer UV-B-Bestrahlung der Nanoemulsionen untersucht (Figur 6).

### Zusammensetzung der Nanoemulsion 9

| | |
|---|---|
| Lecithin | 0,6 % |
| Triglycerid | 0,8% |
| davon: | 90 % C 18:1 und 10 % C 18:2 |
| Borretschöl | 0,2 % |
| Partikeldurchmesser | 60 nm |

### Beispiel 7

### Bestimmung der Genotoxizität von gebrauchtem Fritieröl

Die Mutagenizitätsrate von genotoxischen Substanzen kann zum Beispiel in CHO-Zellen (Chinese Hamster Ovary Cells) bestimmt werden, die geeignete selektierbare Purin- oder Pyrimidin-Auxotrophien aufweisen. Die Genotoxiziät von gebrauchtem Fritieröl kann durch Verkapselung in einer entsprechenden Nanoemulsion (Beispiel Nanoemulsion 10) mit solchen Zellsystemen bestimmt werden.

### Zusammensetzung der Nanoemulsion 10

| | |
|---|---|
| Lecithin | 0,7 % |
| Fritieröl | 1,0 % |
| Partikeldurchmesser | 85 nm |

### Beispiel 8 (nur zur Information)

### Bestimmung der Neurotoxizität von Pestiziden und Herbiziden

Die spezifische Neurotoxizität von Lindan (γ-1,2,3,4,5,6-Hexachlorcyclohexan) kann durch Inkubation von CHEN-Zellen (Chick Embrio Neural Cells) mit einer entsprechenden Nanoemulsion mit eingekapseltem Lindan (Beispiel Nanoemulsion 11) ohne Lösungsschwierigkeiten *in vitro* untersucht werden.

### Zusammensetzung von Nanoemulsion 11

| | |
|---|---|
| Lecithin | 0,38% |
| Caprylsäure-/Carpinsäure-Triglycerid | 0,81 % |
| Lindan | 0,09 % |
| Partikeldurchmesser | 75 nm |

### Beispiel 9 (nur zur Information)

### Bestimmung der Neurotoxizität von Medikamenten

Die spezifische Neurotoxizität von Valium^{®} (Diazepam) kann durch Inkubation von CHEN-Zellen (Chick Embrio Neural Cells) mit einer entsprechenden Nanoemulsion mit eingekapseltem Valium^{®} (Beispiel Nanoemulsion 12) relativ einfach konzentrationsabhängig *in vitro* untersucht werden.

### Zusammensetzung der Nanoemulsion 12

| | |
|---|---|
| Lecithin | 3,32 % |
| Caprylsäure-/Carpinsäure-Triglycerid | 7,20 % |
| Valium^{®} (7-Chloro-1-methyl-5-phenyl-1,4-benzodiazepin-2-on) | 0,36 % |
| Partikeldurchmesser | 80 nm |

### Beispiel 10

### Biotransformation von Hormon-Vorstufen in Zellkulturen

Mit Hilfe von Zellkulturen können komplexe chemische Moleküle wie zum Beispiel Hormone einfach biotechnologisch hergestellt oder gewonnen werden. Dabei können Vorstufen der Moleküle ins Medium gegeben werden, die dann in den Zellen mit spezifischen Enzymen umgewandelt werden und wieder ins Medium abgegeben werden. Mit Hilfe der Nanoemulsionen können lipophile Edukte effizient in diese Zellen eingeschleust werden. Durch einfache Trennmethoden, wie zum Beispiel Dialyse oder "Cross-Flow"-Ultrafiltration, können dann die eingekapselten Edukte leicht von den ins Medium abgegebenen Produkten getrennt werden. Beispielsweise kann 17-Hydroxyprogesteron gezielt mit einer Nanoemulsion in Zwischenzellen von Hodengewebe-Kulturen eingeführt werden. In den Zellen kann dann die Substanz zu 5-α-Androstan-17-β-ol-3-on transformiert werden, die dann wieder ins Medium sekretiert wird.

### Beispiel 11 (nur zur Information)

### Verbesserung der Zelldifferenzierung von primären Hautkeratinozyten mittels Nanoemulsionen

Embryonale Hautkeratinozyten vom Schwein wurden in M199-Medium mit 10 % Serum und mit 0.4 µg/ml Hydrocortison gezüchtet. Die Zugabe von 1 % Nanoemulsion 13 ins Kulturmedium förderte die Differenzierung der Keratinozyten.

### Zusammensetzung der Nanoemulsion 13

| | |
|---|---|
| Lecithin | 0,6 % |
| Caprylsäure-/Caprinsäure-Triglycerid | 0,6 % |
| Vitamin E-acetat | 0,3% |
| Vitamin A-palmitat | 0,1 % |
| Partikeldurchmesser | 45 nm |

### Beispiel 12 (nur zur Information)

### Verwendung einer Nanoemulsion mit Vitamin A und E zur Komplementierung von serumfreien Zellkulturmedien für ein verbessertes Zellwachstum

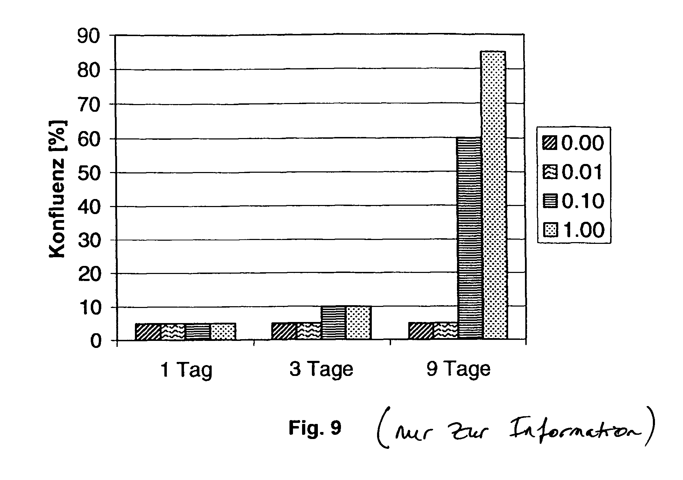

Hybridoma-Zellen, die einen spezifischen Antikörper produzieren, wurden in serumfreien Medium (Cell Culture Technologies) gezüchtet. Das Wachstum der Zellen konnte durch Zugabe der Nanoemulsion 13 verbessert werden (Figur 7).

### Beispiel 13 (nur zur Information)

### Komplementierung einer serumfreien Zellkultur von Fibroblasten mit einer Nanoemulsion zur Erhöhung der Biomasse Produktion

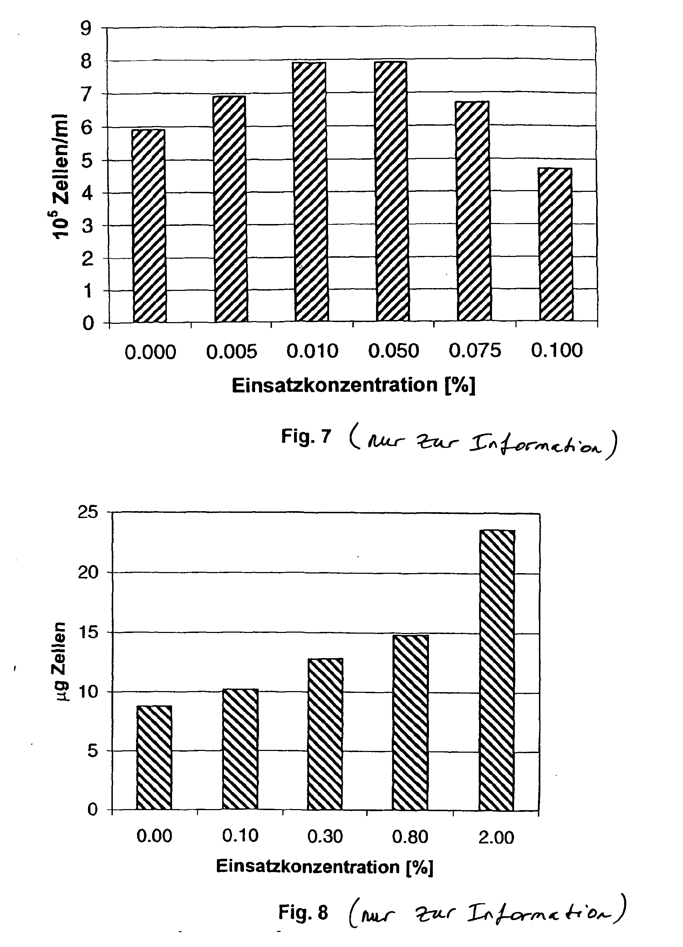

Balb-3T3-Fibroblasten wurden in serumfreien DMEM/F12-(1:1)-Medium (BioConcept) bei verschiedenen Konzentrationen der Nanoemulsion 13 gezüchtet. Die 50 ml-Kulturen wurden nach 9 Tagen geerntet, und es wurde die Biomasse bestimmt (Figur 8).

### Beispiel 14 (nur zur Information)

### Verwendung von Nanoemulsionen mit Vitamin A und E zur Adaptation von Fibroblasten in serumfreien Medium

3T3-Fibroblasten wurden in DMEM/F12-(1:1)-Medium (BioConcept) mit 10 % Serum gezüchtet. Die konfluente Kultur wurde trypsinisiert und in serumfreies DMEM/F12-(1:1)-Medium überimpft, das verschiedene Konzentrationen der Nanoemulsion 13 enthielt. Das Wachstum der Zellen wurde durch Abschätzung der Konfluenz zu verschiedenen Zeiten bestimmt (Figur 9).

### Beispiel 15 (nur zur Information)

### Verwendung von Nanoemulsionen mit ungesättigten Fettsäuren, Vitaminen und β-Carotin zur Erhöhung der Biosynthese von spezifischen Antikörpern in Hybridoma-Zellen in serumfreien Medium

Serumfreie Medien eignen sich besonders gut für die Produktion von Proteinen, da sie keine Fremdproteine enthalten, welche die Isolierung der Produkte beeinträchtigen. In serumfreien Zellkulturen fehlen jedoch häufig essentielle lipophile Stoffe, wie ungesättigte Fettsäuren und Vitamine, die normalerweise im Serum vorhanden sind. Diese Stoffe können nicht ohne weiteres dem serumfreien Medium zugegeben werden, da sie wasserunlöslich sind und nur unkontrolliert dispergiert werden können und eine kleine Bioverfügbarkeit aufweisen. Die Komplementierung solcher Medien mit Nanoemulsionen, die essentielle Lipide enthalten (Beispiel Nanoemulsion 14), kann beispielsweise die Produktion von spezifischen Antikörpern in Hybridoma- Zellen wesentlich verbessern.

### Zusammensetzung der Nanoemulsion 14

| | |
|---|---|
| Lecithin | 0,30 % |
| Triglycerid | 0,50 % |
| davon: | 90 % C 18:1 und 10 % C 18:2 |
| α-Tocopherol | 0,0100 % |
| Vitamin A-acetat | 0,0050 % |
| Linolsäure | 0,0100 % |
| Linolensäure | 0,0100 % |
| β-Carotin | 0,0001 |
| Partikeldurchmesser | 65 nm⁻ |

### Beispiel 16 (nur zur Information)

### Verwendung von Nanoemulsionen zur Erhöhung der Biosynthese von rekombinaten Proteinen

CHO- Zellen (Chinese Hamster Ovary Cells) werden häufig zur Produktion von rekombinaten Proteinen, wie Wachstumsfaktoren, Blutgerinnungsfaktoren und Zytokinen benutzt. Dabei kann die Biosynthese dieser Produkte in serumfreien Medien durch die Zugabe von Nanoemulsionen, die essentielle lipophile Stoffe enthalten (Beispiel Nanoemulsion 13), verbessert werden.

### Beispiel 17 (nur zur Information)

### Verwendung von Nanoemulsionen in Perfusionssystemen

Beispielsweise kann die Nanoemulsion 13 auch zur Erhöhung der Interferon-β- Sekretion von Fibroblasten benutzt werden, die in einem Perfusionssystem gezüchtet werden.

## Patentansprüche

1. Verwendung von keimfreien Nanoemulsionen, welche aus Ölpartikeln, die an ihrer Oberfläche mit einem nicht-toxischen amphoteren Emulgator besetzt sind, in einer wässerigen Phase besteht, wobei die Ölpartikel mindestens einen zu prüfenden oder an die Zellkultur abzugebenden lipophilen Stoff und pro Gewichtsteil Öl 0,65 bis 0,75 Gewichtsteile des amphoteren Emulgators enthalten, zur gezielten und reproduzierbaren Einführung von lipophilen Stoffen in die Zellen von Zellkulturen.

2. Verwendung nach Anspruch 1 zur Bestimmung der Biokompatibilität von lipophilen Stoffen im Zellkultur-Test.

3. Verwendung nach Anspruch 2, **dadurch gekennzeichnet, dass** die lipophilen Stoffe in der Kosmetik eingesetzte Lipide sind

4. Verwendung nach Anspruch 1 zur Bestimmung der Toxizität und/oder der Genotoxizität von lipophilen Stoffen im Zellkultur-Test.

5. Verwendung nach Anspruch 1 zur Biotransformation von lipophilen Stoffen mittels Zellkulturen.

6. Verwendung mit spezifischen lipophilen Stoffen nach Anspruch 1 zur Förderung der Zelldifferenzierung von primären Zellkulturen.

7. Verwendung nach Anspruch 1 zur Komplementierung von Zellkulturmedien mit lipophilen Stoffen, welche das gewichtsmässige Wachstum der Biomasse fördern.

8. Verwendung nach Anspruch 1 zur Komplementierung von Zellkulturmedien mit lipophilen Stoffen, welche die Biosynthese von Stoffen in den Zellen fördern.

9. Verwendung nach Anspruch 1 zur Adaptation von Zellkulturen in serumfreien Medien.

## Claims

1. Use of sterile nanoemulsions composed of oil particles, occupied on their surface with a nontoxic amphoteric emulsifier, in an aqueous phase, the oil particles comprising at least one lipophilic substance intended for testing or for delivery to the cell culture and comprising, per part by weight of oil, 0.65 to 0.75 parts by weight of the amphoteric emulsifier, to introduce lipophilic substances in a targeted and reproducible manner into the cells of cell cultures.

2. Use according to Claim 1 to determine the biocompatibility of lipophilic substances in a cell culture test.

3. Use according to Claim 2, **characterized in that** the lipophilic substances are lipids used in cosmetology.

4. Use according to Claim 1 to determine the toxicity and/or genotoxicity of lipophilic substances in a cell culture test.

5. Use according to Claim 1 to biotransform lipophilic substances by means of cell cultures.

6. Use with specific lipophilic substances according to Claim 1 to promote the cell differentiation of primary cell cultures.

7. Use according to Claim 1 to complement cell culture media with lipophilic substances promoting the growth in weight of the biomass.

8. Use according to Claim 1 to complement cell culture media with lipophilic substances promoting the biosynthesis of substances within the cells.

9. Use according to Claim 1 to adapt cell cultures in serum-free media.

## Revendications

1. Utilisation de nano-émulsions stériles, constituées de particules d'huiles en phase acqueuse, occupées en surface par un émulsifiant amphotère non toxique, les particules d'huile contenant au moins une substance lipophile à examiner ou à céder à la culture de cellules et 0,65 à 0,75 de parties pondérales d'émulsifiant amphotère par partie pondérale d'huile pour l'introduction ciblée et reproductible de substances lipophiles dans les cellules de cultures de cellules.

2. Utilisation suivant la revendication 1 pour la détermination de la biocompatibilité de substances lipophiles dans le test de culture de cellules.

3. Utilisation suivant la revendication 2, **caractérisée en ce que** les substances lipophiles sont des lipides employés en cosmétique.

4. Utilisation suivant la revendication 1 pour la détermination de la toxicité et/ou de la génotoxicité de substances lipophiles dans le test de culture de cellules.

5. Utilisation suivant la revendication 1 pour la biotransformation de substances lipophiles au moyen de cultures de cellules.

6. Utilisation avec des substances lipophiles spécifiques suivant la revendication 1 pour favoriser la différenciation des cellules de cultures primaires.

7. Utilisation suivant la revendication 1 pour la complémentation de milieux de cultures de cellules en substances lipophiles favorisant la croissance de la biomasse au niveau pondéral.

8. Utilisation suivant la revendication 1 pour la complémentation de milieux de cultures de cellules en substances lipophiles favorisant la biosynthèse de substances dans les cellules.

9. Utilisation suivant la revendication 1 pour l'adaptation de cultures de cellules dans des milieux libres de sérum.
